# EUROPEAN PATENT SPECIFICATION

(11) **EP 4 063 253 B1**
(45) Date of publication and mention of the grant of the patent: **14.08.2024**
(21) Application number: 21202308.9
(22) Date of filing: 12.10.2021
(51) Int. Cl.: B63B 22/00

(54) **A BUOY FOR CHECKING CONDITION OF A PERSON OR AN OBJECT TIED TO THE SAID BUOY**
BOJE ZUR PRÜFUNG DES ZUSTANDS EINER PERSON ODER EINES AN DIE BOJE GEBUNDENEN GEGENSTANDS
BOUÉE PERMETTANT DE VÉRIFIER L'ÉTAT D'UNE PERSONNE OU D'UN OBJET ATTACHÉ À LADITE BOUÉE

(30) Priority: 26.03.2021 SI 202100061
(43) Date of publication of application: 28.09.2022
(73) Proprietor: Seavision d.o.o., 1356 Dobrova (SI)
(72) Inventor: Peklenk, Anze, 1356 Dobrova (SI); Podbevsek, Andrej, 1241 Kamnik (SI); Jasarov, Matija, 3000 Celje (SI); Tomazevic, Matic, 1292 Ig (SI)
(74) Representative: Patentni Biro AF d.o.o.

(56) References cited:
- AT-A2- 517 793
- CN-A- 110 203 333
- US-A1- 2015 116 496
- US-B1- 6 383 045

## Description

### Field of the invention

The present invention belongs to the nautical field, specifically to the field of buoys for tying vessels. The invention relates to a buoy for checking the condition of a person or an object tied to the said buoy.

### Background of the invention and the technical problem

A buoy is a floating structure used for different purposes. Several different types of buoys are available: a communication buoy, a navigation buoy, a sonar buoy, a markation buoy, a weather buoy and a diving buoy, wherein they can be anchored or free-floating. A buoy can be used for marking vessel quays, especially outside marina. All buoys have a common characteristic - they mark a certain location, and they can emit and receive data important for navigation, measurements or communication.

The buoy attached to a vessel mooring or in most cases to a vessel, which is anchored with an anchor, it is important to monitor the condition of the mooring as well as the condition of the vessel's anchor. While the state of the mooring (presence of vessel) can be monitored with the solution described in the European patent EP 2 531 981 B1, it does not enable monitoring of the anchor condition or any other events under the water surface. Some other solutions can only monitor the vessel location, which is not important for the underwater condition of anchor, since its micro location can change in such a manner that the anchor may become entangled or stuck. The technical problem, solved by the said invention, is construction of a buoy, which will enable checking the condition of person or object fastened (tied or attached) to the said buoy.

For this purpose underwater drones or cameras, such as described in the patent application US20050036031, may be used, however, they must be monitored by the user at all times otherwise important data is lost in important moments. The preparation of currently known underwater drones is time-consuming. Additionally, they need to locate the desired object or the person in the water in order to allow monitoring. When the drone is back on land or on vessel the monitoring of the person or the object is lost. The camera, as described in the patent application US20050036031 is intended mostly for observing sea life and is connected via cable to a specific device, which requires continuous monitoring. It does not display the object's or person's position and it is very time-consuming for preparation. When the water depth is changing, it is also important to monitor and unwind the cable in order to maintain same position. The present invention solves listed shortcomings and disadvantages.

### State of the art

The patent application US2015116496A1 describes an anchor, equipped with a camera, a sensor and a light. The system connected to the camera, sensor and light on the anchor guarantees anchor monitoring in real time and enables notifications regarding the anchor state.

Patent US9729253 discloses a system with an underwater housing, which comprises an underwater wireless communication module, connected to the camera and an above-water housing with an above-water wire communication module, wherein both communication modules communicate via a cable. Camera captures photographs or video, which is forwarded via modules to a mobile device to monitor the conditions underwater.

Patent application AU20141 00760A describes a safety buoy for use in marines, which has an electronic control unit and a radio transmitter that launches communication with the satellite network and sends out a distress call when activated with the electronic unit.

Document AT517793 describes a device for optical control of water bodies, especially bodies underwater, the said device being provided with an attached buoy, wherein the rope of the buoy has a waterproof housing with installed cameras directed in all directions, along with a compass. The device can also include a sensor for determining camera position. An image can be captured with a trigger button via a transponder and suitable electronics.

Patent application US5803780 discloses a buoy for marking, which can be used to simply mark a location at the sea bed. The buoy comprises a control mechanism, which releases a weight by using the optimal rope amount.

Patent application US7034680 describes a device for monitoring anchor or anchor rope, which includes a measuring device, which uses one or more sensors to determine a status of one or more points on the anchor or the anchor rope with regards to the vessel and generates a signal to trigger an alarm when it deviates from the pre-set values.

Patent application CA2088088 relates to a buoy for marking a location of an underwater object, especially a sunken vessel's anchor. A spherical buoy housing is connected via a nylon rope and an attachment to the anchor before it submerges. The length of the unwinding rope is controlled by a mechanism, so that the location of the buoy is not changed due to waves or other water movement, which enables reliable marking of the location of the anchor under water.

The present invention differs from the above solutions in its construction.

The most similar solution is described in patent application US2015116496, which relates to a system associated with the camera, sensor and/or light-equipped anchor provides for real-time monitoring of the anchor and for an alert system regarding the status of the anchor. This solution uses a buoyant float on which transmitter, GPS positioning system, cable reel with a cable and a battery are mounted, wherein a camera is lowered away from the float due to unwinding of the cable from the cable reel. The disadvantage of this solution is low protection of the key components for operation of the device, as they are prone to come into contact with water leading to malfunction.

### Description of the solution of the technical problem

The essence of the buoy according to the invention is in that it comprises a casing, which floats on the water surface, a capsule with camera and lights, which can be separated from the casing with a UTP cable and lowered down to an anchor, an object or a person. The wire rope, which is connected to the lower part of the capsule can be connected to any anchor, fixed object on the sea bed or it can be attached to a person (diver) with the wire rope. The camera captures images and/or video and sends data via UTP cable to the processor, and the processor emits data via wireless network (WiFi or similar) to the mobile application installed on the user device, which is preferably a smart phone, a tablet, computer or a similar device. The user can visually check the condition of the anchor and simultaneously monitor the anchor position via one or several satellite systems for global navigation (GNSS), e.g. GALILEO, GPS, GLONASS, BEIDOU, and the position is displayed graphically and numerically on an application on mobile telephone, tablet or computer. The buoy sends out images and video to the application, as well as telemetric data on battery condition, turning on and off the camera and lights, and sends out data to the server online or in a computer cloud, making the data, images and video available via any device connected to the internet.

The buoy according to the invention comprises:
- the casing with an upper part with solar cells for ensuring energy for function and a lower part;
- a Watertight and/or waterproof chamber for key functional components of the buoy, wherein said chamber is installed in the interior of the buoy casing and houses a battery, a processor, the positioning module for determining geographic position via one or more systems for global navigation (GNSS, e.g. GALILEO, GPS, GLONAS, BEIDOU), a radio WiFi transmitter and receiver and a module for data transfer via network of mobile communication (e.g. LTE, 5G);
- a capsule with the camera and at least one light, preferably more lights; most preferably the lights are a combination of IR and LED lights, wherein the capsule is connected to the bottom part of the casing in a separable manner;
- an UTP/Ethernet cable, which connects to the buoy capsule with the casing of the buoy, where the cable is connected to the processor, wherein the cable unwinds and coils with a rotating mechanism, comprising:
   ∘ a reel, installed on an aluminium shaft, which acts as a rotating rotation axis, and inside which the UTP cable is installed, wherein the aluminium shaft is installed in the casing with holders up to a sliding ring;
   ∘ the sliding ring, which consists of rotating and non-rotating rotating part, and is arranged to accept electrical circle from the battery to the capsule and a video signal from the camera to the processor, wherein the UTP cable is set up through an aluminium shaft to the rotating part of the slide ring and via sliders in the slide ring to the non-rotating part of the slide ring, installed in the chamber;
   ∘ a bushing or a slide bearing installed on the aluminium shaft for ensuring unlimited rotation of the said shaft, and
   ∘ a retractable tension spring, for ensuring automatic unwinding and winding the UTP cable on the reel;
- a wire rope with one end attached to the bottom part of the capsule, the other end being free and preferably equipped with a ring for attaching an anchor or any other object or a person.

The buoy casing is primarily made from ecologically processed, strong and resistant plastic mass, and both parts are assembled with threads and seals. The buoy is designed to preserve buoyancy even in case it submerges, because the watertight chamber for the key components in the buoy interior is an airtight sealed space and the air inside the buoy maintains it above water.

The upper part of the buoy can be partially transparent and can additionally comprise signal lights in case of danger or anchor movement. The upper part may also comprise solar cells for charging the battery, which is installed in the waterproof chamber for key components, wherein the solar cells can be installed on the casing, but are preferably provided in the interior under the transparent material of the upper part of the casing. The lower part of the buoy has an opening at the bottom for the UTP/Ethernet cable. The bottom part of the buoy casing can have small holes at the bottom part, which enable draining of excess water, which remains in the buoy when lifted. The bottom part of the casing is designed to have a movable capsule with the camera and lights, or the casing is in its bottom part designed as a cavity, which can partially hide the capsule with the camera and lights.

The capsule can be separated from the buoy casing by unwinding the cable using the rotating mechanism, which operates in such a manner that upon attaching a load on the wire rope the capsule is lowered due to gravity, while in case the load is removed from the wire rope the UTP cable is automatically wound on the reel due to the tensile spring, which is attached at the end of aluminium shaft in a fixed manner. The latter is attached to the two holders and together with the bushing enables the reel to be stable and attached in the interior of the buoy casing, wherein the bushing enable shaft rotation of the shaft without traction. The sliding bearing is made from highly resistant plastics and ensures mechanical stability with lowest coefficients of traction without additional greasing and is resistant to sea water. One end of the aluminium shaft is attached to the sliding bearing, and the other part is installed to the spring with constant force in a fixed manner. The spring is made from a coiled steel wire, and its coils enable stretching and automatic contraction. In case the load from the wire rope is removed, the UTP cable is automatically wound back on the reel. In case the cable retracts, i.e. it is pulled out of the buoy casing, the pulling force must be higher than the force of the spring. This causes that the UTP cable is constantly tensed and that the buoy above the anchor is always in the same position above it. Even in the case of waves the spring is loosened and automatically winds the cable back to the reel. The holders of the aluminium shaft are attached with screws to the bottom part of the buoy casing, said holders being made from stainless steel.

The watertight and/or waterproof chamber for key components for buoy operation is installed in the interior of the buoy casing, preferably the chamber of key components is made from two parts and the bottom part is with screws attached to the bottom part of the buoy casing, and the cover is tightly attached with screws and seals to the bottom part of the chamber of key components, which are the battery, the processor, the positioning module for determining geographic position with one or more systems GNSS, radio WiFi transmitter and receiver and a module for data transfer vial networks of mobile communication (LTE, 5G). On the inner part at the side of the chamber cover an opening is provided, through which the non-rotating part of the slide ring is installed, said non-rotating part leading the UTP cable from the processor via the rotating mechanism to the capsule.

The watertight capsule is made from two parts, i.e. the upper part and bottom part of the capsule, which are connected to each other with screws and a seal. The bottom part, from the internal side, has a space for the camera and lights, preferably which are preferably IR and LED lights. The bottom part has a suitably designed opening and sealed transparent part so that the camera can take images, while the lights emit light through the transparent material. The entire capsule is covered in rubber (except the opening for camera and lights), which absorbs hits and protects the plastic part of the capsule mould from external influences. The external part of the bottom part of the capsule has an attachment element for the wire rope, which can be approximately 2 m long, and its free end is provided with a ring or any other suitable element to which an anchor or any other object or a person in the water can be attached or tied.

The camera in the capsule has the UTP cable that runs from the buoy casing, which fuels the camera with PoE (Power over Ethernet) electric current, which is used for sending image and video signal from the camera to the processor, which can send the data via WiFi to the application and via the module for data transfer via mobile network on the server. The camera is controlled via the cable with the processor and the application. The camera records in high resolution, and has an option of recording and saving individual photographs. All videos and images can be archived in the processor, the application and on a server. The camera enables visual control/monitoring of a certain object or a person underwater in real time. Preferably the camera has three modes of function. If the LED lights are on, the camera functions as usually, and if only IR lights are on, it operates in a night mode, wherein the camera can operate even with lights off if the visibility is suitable and enough natural light is available. The lights are charged with the UTP cable, and their function is controlled with an application on mobile devices or remotely via the server. Preferably, the application allows to choose turning on or off lights, which is communicated to the processor via a wireless connection, which forwards the information via the UTP cable to the camera in order to follow the processor commands. The camera is suitably connected to the lights so that it can adjust their function or light.

The application performs the following steps:
- Displaying video or image view of the anchor,
- Displaying anchor location on a map,
- Enabling adjustment of light and camera status on the buoy as described above,
- Enabling geofencing of the anchor up to 1 to 20 metres, which means that if the anchor deviates from the pre-set limits, the alarm is triggered thus notifying the user the anchor has moved outside the certain area, wherein the alarm can be auditory, image or in any other suitable alarm, and
- archiving the image and video content by storing data on a suitable server.

For the preferred use of the buoy in monitoring the anchor condition and checking the anchoring at the same location, the application is used to set a geolocation, defined with a circle from 1 to 20 metres. In the moment the position is set it is archived by the positioning module, and fencing is implemented by a programme equipment with the processor. The drawn circle of the limitation is displayed on the map, and any potential additional movement of the buoy is monitored by the positioning module after setting up the position. If the anchor leaves the pre-set circle limit the alarm s triggered, and the processor acts accordingly. The buoy efficiently monitors the anchor movement on its own, its movement and together with capturing images and/or video enables safer anchoring of any vessel in any water, preferably in the sea, where movements, waves and tide are important factors for movement of vessels and anchors.

The process of using the buoy according to the invention comprises the following steps:
a) Registration into the application with a username and a password,
b) Connecting with the buoy or a specific buoy, if there are more, and naming the buoy;
c) Displaying location of the selected buoy on a map;
d) Connecting an anchor or an object tied to the buoy and lowering the anchor or the object into water, which causes automatic lowering or unwinding of the cable from the buoy;
e) Once the anchor or the object is d on sea bed or at a certain depth, the unwinding stops or it winds back and lowers further via the self-winding system if needed, depending on the waves or movement of the anchor or the object;
f) If needed activation of lights and capturing image or video of object, connected to the buoy;
g) Displaying image or video, wherein the lights can be controlled or adjusted (IR, LED and intensity) as described above;
h) If desired or optionally, allowing magnification, storing the images to a smartphone and access to gallery;
i) Optional setting of geographic limits (geofencing) from 1 to 20 metres, which generates a circle around the buoy position;
j) Possible triggering of the alarm if anchor moves out of the set circle;
k) If desired, turning off a camera and/or lights;
l) If necessary, lifting the anchor or object from the sea bed towards sea surface, which causes automatic winding of the UTP cable using the self-winding system;
m) Lifting the buoy out of the water.

The buoy according to the invention can be used to monitor underwater activity, especially on sea, lake or river bed. It is primarily intended for observing and monitoring the condition of the anchor, which is tied to the buoy, ensuring safe anchoring of the vessel anywhere in the sea, lake or river. Simultaneously, the buoy can be used to observe underwater life, a visual or image depiction of sea, lake or river bed. Nonetheless, the buoy according to the invention improves the safety of divers, since it can constantly monitor their dive and/or work and in case of problems ensures quick reactions.

The buoy for checking the condition of person or object tied to the said buoy according to the invention will be described in further detail based one exemplary embodiments and figures, which show:
- Figure 1: the buoy according to a possible embodiment with the capsule in the starting position.
- Figure 2: the buoy according to a possible embodiment with a lowered capsule.
- Figure 3: the interior of the buoy casing.
- Figure 4: a section of the rotating mechanism.
- Figure 5a: a section of the rotating mechanism and the chamber housing the key components.
- Figure 5b: a section of the rotating mechanism, the chamber housing the key components and the capsule.
- Figure 6: Use of the buoy for marking location and monitoring the diver.
- Figure 7: Use of the buoy for marking location and monitoring the sea bed condition.
- Figure 8: Use of the buoy for marking location and monitoring the anchor status used to anchor the vessel.

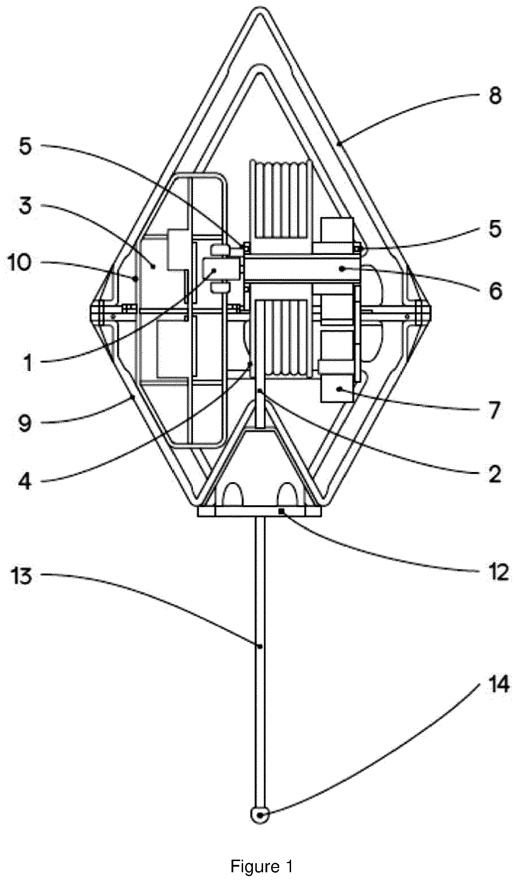

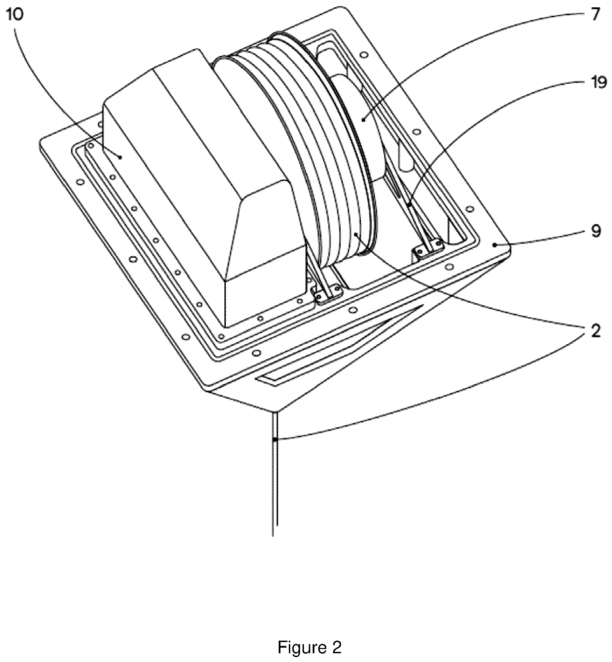

A possible embodiment of the buoy is shown Figures 1 and 2 and comprises:
- A casing, which has an upper part 8 with solar cells 18 for guaranteeing energy for function and bottom part 9, wherein the casing in the upper part is made from see-through plastic material and the solar cells 18 installed on the inner side of the see-through upper part 8 of the buoy casing;
- A watertight and/or waterproof chamber 10 for key components 3 for buoy function, which is installed in the casing interior 8, 9 of the buoy and which has installed battery, processor, positioning module, WiFi transmitter and LTE module, wherein the positioning module is any GNSS.
- Capsules 12 with a camera 15 and lights 16, 17, primarily the lights are a combination of IR 16 and led lights 17, wherein the capsule 12 is separable connected to the bottom part of the casing 9;
- A UTP/ethernet cable 2, which connects the capsule 12 to the buoy casing 8, 9 which is connected to the processor, wherein the cable 2 unwinds and coils with the help of a rotating mechanism, comprising:
   ∘ A reel, installed to the aluminium shaft 6, which acts as a rotating axis, and has installed a UTP cable 2, wherein the aluminium shaft 6 is installed in the casing with the help of the pylons 19 up to the slide ring 1;
   ∘ A slide ring 1, which consists of rotating and non-rotating part, and it is adjusted to accept electrical circle from the battery to the capsule 12 and a video signal from the camera to the processor, wherein the UTP cable 2 is set up through an aluminium shaft 6 to the rotating part of the slide ring 1 and via sliders in the slide ring to the non-rotating part of the slide ring, installed in the chamber 10;
   ∘ A bushing 5 or slide bearing installed to the aluminium pipe 6 for guaranteeing undisturbed rotating of the said pipe 6, and
   ∘ A retractable tensile spring 7, which enables automatic reeling and winding the UTP cable 2 on the reel;
- A wire rope 13 attached to the bottom part of the capsule 12, and the free part of the wire rope is primarily equipped with a hoop 14 for attaching an anchor or any other object or person.

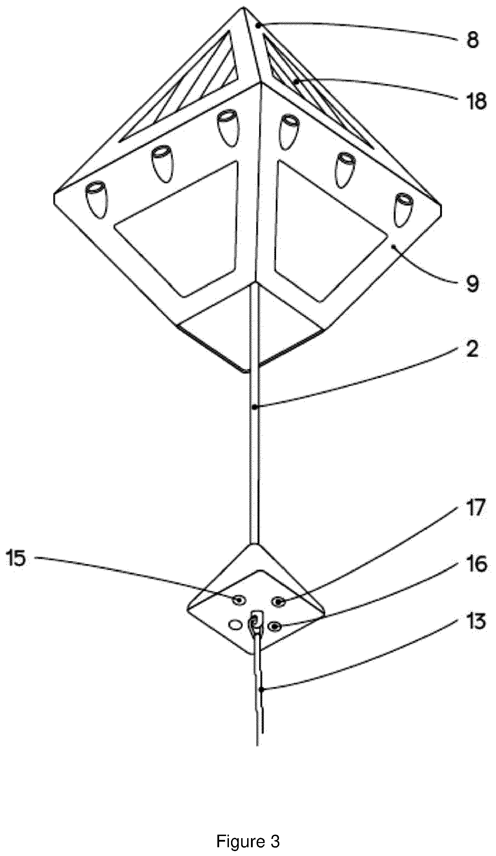

The buoy casing 8, 9 is made from ecologically processed, strong and resistant plastic mass, and both parts are assembled with threads and seals. The upper part 8 also comprises solar cells 18 for charging the battery, which is installed in the waterproof chamber 10 for key components 3, wherein the solar cells 18 are installed below the transparent material of the upper part 8 of the housing. The bottom part 9 of the buoy casing has an opening at the bottom, through which the UTP/Ethernet cable 2 runs, and at the same time the bottom part has a designed cavity to hide the capsule 12 with the camera 13 and lights 16, 17. The capsule 12 can be lowered as shown in Figure 3, wherein it remains connected to the buoy with the UTP cable 2.

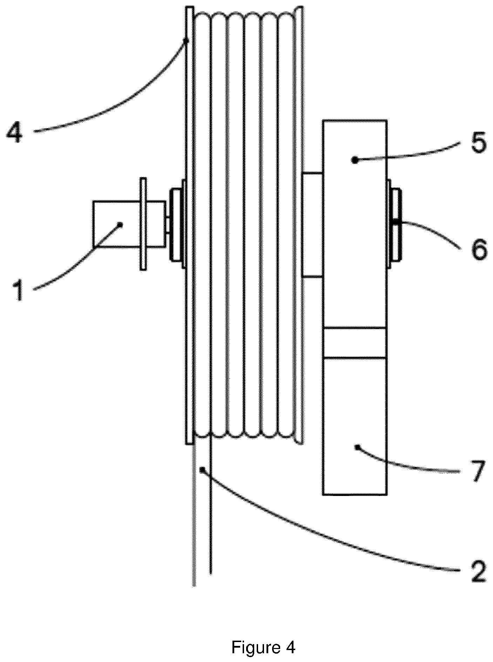

Figure 4 displays the section of rotating mechanism, comprising:
- the reel, installed on an aluminium shaft 6, inside which the UTP cable 2 installed,
- the slide ring 1, which comprises a rotating and a non-rotating part, wherein the latter is installed in the chamber 10,
- the bushing 5 or a slide bearing installed on the aluminium shaft 6 for ensuring unlimited rotation of the said shaft, and
- the self-winding tension spring 7.

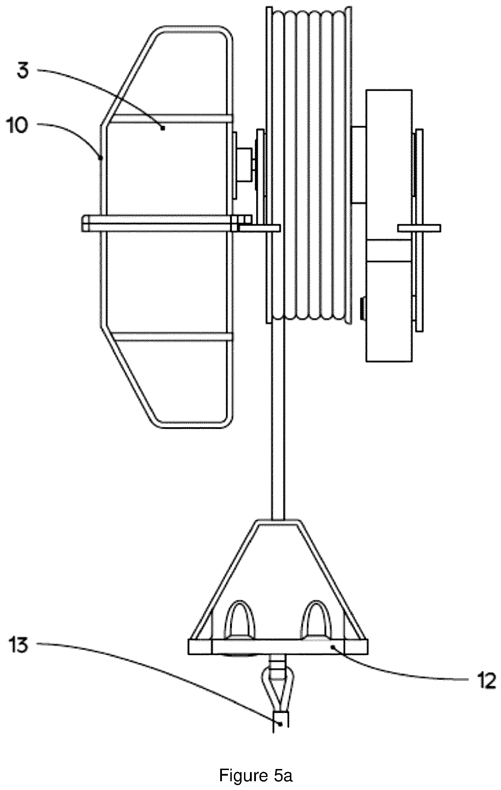

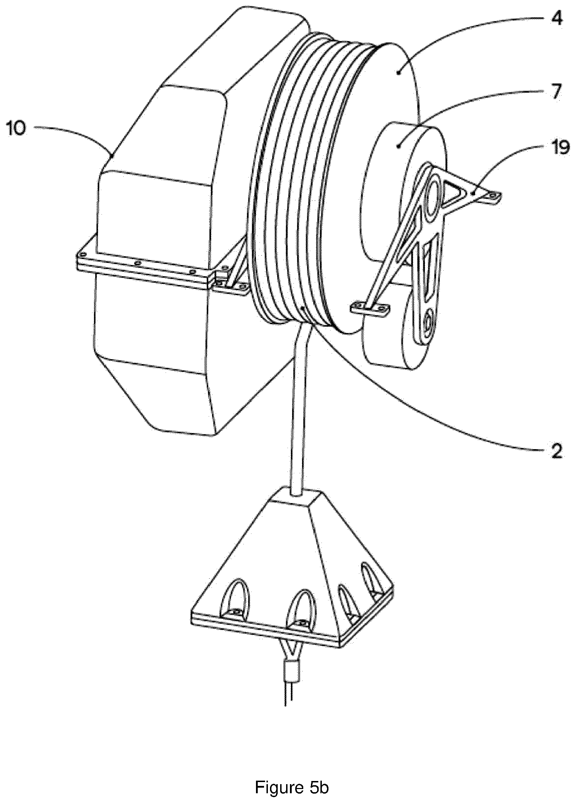

Figures 5a and 5b in addition to the rotating mechanism also the capsule 12 and the chamber 10 are shown, while the non-rotating part of the slide ring is not visible.

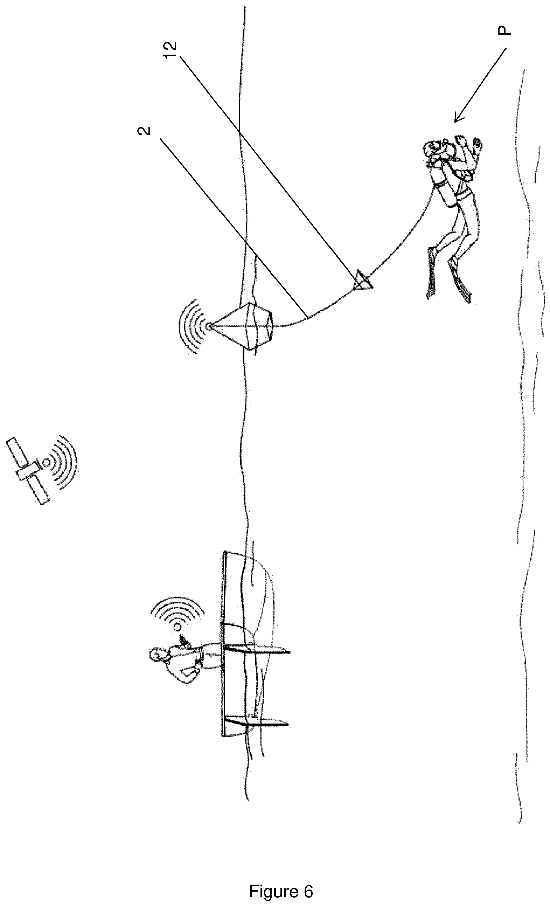

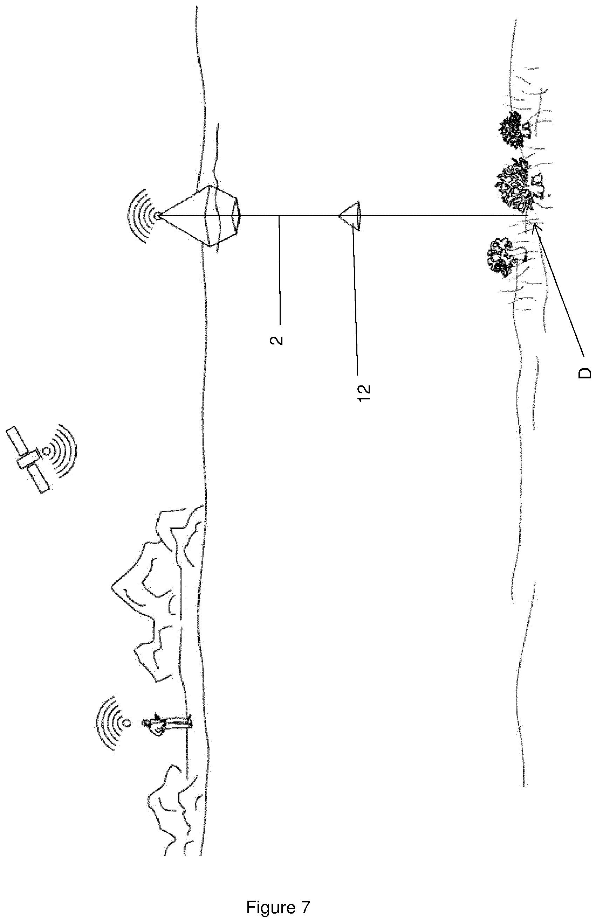

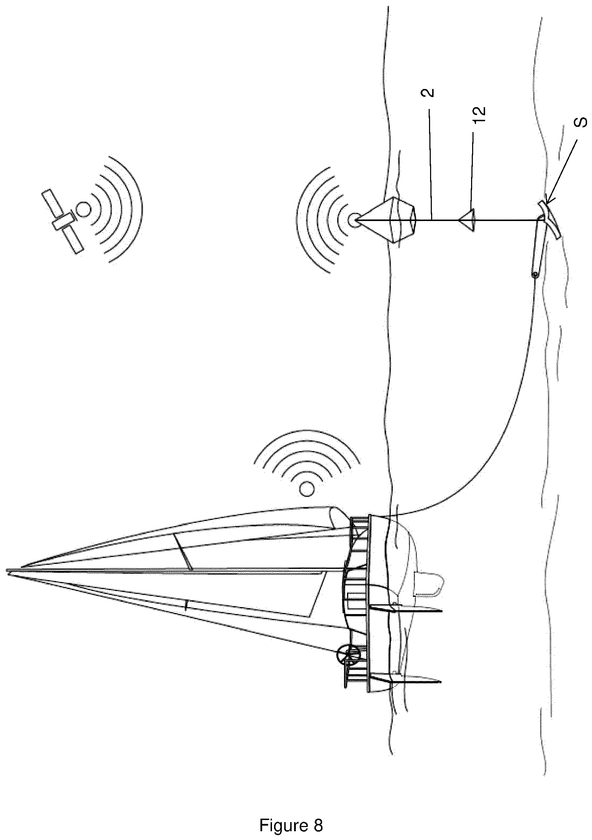

Figures 6 to 8 show some possible ways of use, e.g. use of the buoy for marking location and monitoring the condition of a diver P, a location at sea bed D or an anchor S, which is used to anchor a vessel. In all cases the wire rope of the capsule 12 is tied or attached to the diver P, and the anchor S or wire rope is attached to the object at the sea bed, which causes separation on the capsule 12 from rest of the buoy, i.e. the capsule 12 is located lower than the buoy floating on the water surface. The UTP cable 2, which connects the buoy with the lowered capsule 12 enables transfer of energy for operation of the camera and lights as well as data transfer of capturing images or video with camera, and also for controlling the components of the capsule 12.

## Claims

1. A buoy for checking condition of a person or an object tied to the said buoy, wherein the buoy comprises:
- a casing (8, 9), which is arranged to float on sea surface, and
- a capsule (12) with a camera (15) and lights (16, 17), which can be separated from the casing (8, 9) with a UTP cable (2) and is arranged to be lowered towards an anchor, an object or a person, wherein the cable (2) unwinds and winds using a rotating mechanism, which comprises a sliding ring (1) comprising a rotating and non-rotating part which enable unwinding of the UTP cable (2), which ensures smooth transfer of electric energy and video signal via UTP cable (2) from a camera (15) to a watertight and/or waterproof chamber (10) for housing key components (3) and in vice-versa,
wherein the camera (15) is arranged to capture images and/or video and send data via the UTP cable (2) to a processor, said processor being arranged to send data via wireless network (WiFi or similar), to a mobile application installed on a user device, which is preferably a smart phone, a tablet, computer or a similar device,
**characterised in that**:
- the casing (8, 9) comprises:
∘ an upper part (8) shaped as a pyramid and provided with solar cells (18) for ensuring energy for function, and
∘ a lower part (9) shaped as a truncated pyramid;
- in the interior of the casing (8,9) the watertight and/or waterproof chamber (10) for key components (3) for operation of the buoy is installed, wherein inside the chamber a battery, the processor, a positioning module for determining geographic position via one or more systems for global navigation (GNSS, e.g. GALILEO, GPS, GLONAS, BEIDOU), radio WiFi transmitter and a receiver and a module for data transfer via network of mobile communication (e.g. LTE, 5G) are installed,
- the bottom part (9) of the buoy casing has an opening at the bottom, through which the UTP/Ethernet cable (2) runs, and the bottom part (9) is designed to allow installation of the movable capsule (12) with the camera (15) and lights (16, 17); or the casing (9) is in the bottom part designed as a cavity, which can at least partially enclose the capsule (12) with the camera (15) and lights (16, 17),;
- the UTP/Ethernet cable (2), which connects the capsule (12) to the buoy casing, which is connected to the processor, wherein the cable (2) unwinds and coils with the help of a rotating mechanism installed adjacent to the chamber (10), said rotating mechanism comprising:
∘ a reel, installed to the aluminium shaft (6), which acts as a rotating axis, and has installed a UTP cable (2), wherein the aluminium shaft (6) is installed in the casing with the help of the pylons (19) up to a slide ring (1);
∘ the slide ring (1), which consists of rotating and non-rotating part, and it is adjusted to accept electrical circle from the battery to the capsule (12) and a video signal from the camera (15) to the processor, wherein the UTP cable (2) is set up through an aluminium shaft (6) to the rotating part of the slide ring (1) and via sliders in the slide ring (1) to the non-rotating part of the slide ring, installed in the chamber (10);
∘ Bushing (5) or slide bearing installed to the aluminium pipe (6) for guaranteeing undisturbed rotating of the said pipe, and
∘ Retractable tensile springs (7), which enables automatic reeling and winding the UTP cable (2) on the reel;
- a wire rope (13) attached to the bottom part of the capsule (12), and the free part of the wire rope (13) is equipped with a hoop for attaching an anchor (S) or any other object (D) or person (P).

2. The buoy according to claim 1, **characterised in that** the positioning module is one or several systems for global navigation (GNSS) selected in a group consisting of GALILEO, GPS, GLONAS, BEIDOU and any combination thereof.

3. The buoy according to any of the previous claims, **characterised in that** the casing (8, 9) of the buoy is made from ecologically processed, strong and resistant plastic mass, and that both parts (8,9) of the casing are assembled with threads and seals.

4. The buoy according to any of the previous claims, **characterised in that** the upper part (8) of the buoy is partially transparent and can additionally include signal lights in case of danger or movement of anchor, and **in that** solar cells (18) can be installed on the casing, preferably the solar cells (18) are installed in the interior of the casing underneath a transparent material of the upper part (8) of the casing.

5. The buoy according to any of the previous claims, **characterised in that** the capsule (12) can be separated from the buoy casing (8, 9) by unwinding the cable (2) with the rotating mechanism, which acts by attaching to the weight on the wire rope (13) and due to gravity the capsule (12) moves downwards by unwinding cable (2), while when weight is removed on the wire rope (13) the cable (2) is automatically winds on the reel due to function of the tensile spring (7), which is fixed attached at the end of aluminium shaft (6), attached to the two pylons (19) and along with the bushing (5) enables the reel to be stable and attached in the interior of the buoy casing, and the bushing (6) enables shaft rotating without traction, and end of the aluminium shaft is attached to the slide bearing, and the other part is fixed installed to the retractable spring (7) with constant force that is made from coiled steel ribbon, and the coils of the spring enable stretching and automatic contraction.

6. The buoy according to any of the previous claims, **characterised in that** the watertight and/or waterproof chamber (10) for key components for buoy function is installed in the interior of the buoy casing, and predominantly the chamber (10) of key components is in two parts and the bottom part (11) with screws is reeled in the bottom part (9) of the buoy casing, and the cover is tightly attached with screws and seals to the bottom part of the chamber of key components, such as battery, processor, positioning module for determining geographic position via one or more systems GALILEO, GPS, and/or GLONAS, radio WiFi transmitter and receiver and a module for data transfer vial networks of mobile communication (LTE, 5G), and the inner part by the side of the chamber cover has an opening, which has a non-rotating part of the slide ring (1), which leads the UTP cable (2) from the processor via the rotating mechanism to the capsule (12).

7. The buoy according to any of the previous claims, **characterised in that** the watertight capsule (12) is made from the upper part and bottom part of the capsule (12), which are assembled with screws and a seal, wherein the bottom part has a suitably designed opening and a sealed transparent part so that the camera (15) can capture images, while the lights (16, 17) emit light through the transparent material.

8. The buoy according to any of the previous claims, **characterised in that** the entire capsule (12), except for the opening for the camera (15) and lights (16, 17), is covered in rubber, which absorbs hits and protects the plastic part of the capsule (12) mould against external influences.

9. The buoy according to any of the previous claims, **characterised in that** it is arranged to use the processor and the application to control the camera (15) via the UTP cable (2), said camera (15) enabling visual control/monitoring of a certain object (S, D) or a person (P) underwater in real time, wherein all recordings and images can be archived in the processor, the application and on a server.

10. The buoy according to any of the previous claims, **characterised in that** the camera has three modes of function:
- If the LED lights (17) are on, the camera (15) functions normally,
- if only IR lights (16) are on, the camera (15) operates in a night mode,
- the camera (15) operates even when the lights are off, if visibility is suitable and enough natural light is available.

11. The buoy according to any of the previous claims, **characterised in that** the application performs at least the following steps:
- Displays video or image view of the anchor (S),
- Displays anchor (S) location on the map,
- Enables adjusting conditions of light (16, 17) and camera (15) on the buoy as described above,
- Enables the geofencing of the anchor (S) up to 1 to 20 metres, which means if the anchor (S) deviates from the pre-set limits, the alarm goes off, notifying the user the anchor has moved outside the certain area, wherein the alarm can be auditory, image or in any way suitable and
- It archives the image and video content by storing to the suitable server.

12. The buoy according to any of the previous claims, **characterised in that** the application is arranged in such a manner that for preferred use of the buoy in monitoring the anchor (S) condition and checking the anchoring (S) at the same location, the application is used to set a geolocation, thus defining a circle with a radius from 1 to 20 metres, wherein in the moment the position is set, is the position is archived by the positioning module, and fencing is implemented by a software with the processor thus drawing a limitation circle on the map, wherein any potential additional movement of the buoy is monitored by the positioning module after setting up the position, and wherein the anchor oversteps the pre-set circle limit the alarm is arranged to be triggered, wherein the processor is arranged to act accordingly.

13. A process of using the buoy according to any of the preceding claims, said process comprising the following steps:
a) Registration into an application with a username and password;
b) Connecting with the buoy or a specific buoy, if there are more, and naming the buoy;
c) Displaying location of buoy on map;
d) Connecting an anchor (S) or object (D) or person (P) tied to the buoy and descend into water, which causes automatic descending or unwinding of cable from buoy;
e) Once the anchor (S) or object (D) or person (P) is placed on sea bed or at certain depth the unwinding stops or it coils back and lowers further via self-coiling system if needed, depending on the waves or movement of anchor or object;
f) Optionally, activation of light and capturing image or video of object, connected to buoy;
g) Displaying image or video with the option of managing and adjusting lights (IR, LED and intensity) as described above;
h) optionally, the option of enhancing and archiving images to the smartphone and access to gallery;
i) Optional setting of geographic limits (geofencing) from 1 to 20 metres, drawing a circle around the buoy position;
j) Possible alarm triggering when anchor moves out of the set circle;
k) optionally, turning off a camera (15) and/or lights (16, 17);
l) optionally, lifting the anchor or object from the sea bed towards sea surface, which is managed by automatically coiling the UTP cable (2) with the assistance of self-coiling system (7); and
m) lifting the buoy from the water.

## Patentansprüche

1. Boje zur Prüfung des Zustands einer Person oder eines an die Boje gebundenen Gegenstands, wobei die Boje umfasst:
- ein Gehäuse (8, 9), das angeordnet ist, um auf der Meeresoberfläche zu schwimmen, und
- eine Kapsel (12) mit einer Kamera (15) und Leuchten (16, 17), die mit einem UTP-Kabel (2) vom Gehäuse (8, 9) getrennt werden kann und angeordnet ist, um in Richtung eines Ankers, eines Gegenstands oder einer Person abgesenkt zu werden, wobei das Kabel (2) mithilfe eines Drehmechanismus, der einen Gleitring (1) umfasst, der ein rotierendes und ein nicht rotierendes Teil umfasst, welche das Abwickeln des UTP-Kabels (2) ermöglichen, ab- und aufgewickelt wird, was eine reibungslose Übertragung von elektrischer Energie und einem Videosignal über UTP-Kabel (2) von einer Kamera (15) zu einer wasserundurchlässigen und/oder wasserdichten Kammer (10) zum Unterbringen von Schlüsselkomponenten (3) und umgekehrt gewährleistet,
wobei die Kamera (15) angeordnet ist, um Bilder und/oder Videos aufzunehmen und Daten über das UTP-Kabel (2) an einen Prozessor zu senden, wobei der Prozessor angeordnet ist, um Daten über ein drahtloses Netzwerk (WiFi oder ähnliches) an eine auf einer Benutzervorrichtung installierte mobile Anwendung zu senden, die vorzugsweise ein Smartphone, ein Tablet, ein Computer oder eine ähnliche Vorrichtung ist,
**dadurch gekennzeichnet dass**:
- das Gehäuse (8, 9) umfasst:
∘ ein Oberteil (8), das die Form einer Pyramide aufweist und mit Solarzellen (18) zum Sicherstellen der Energie zur Funktion versehen ist, und
∘ ein Unterteil (9), das die Form eines Pyramidenstumpfes aufweist;
- im Inneren des Gehäuses (8,9) die wasserundurchlässige und/oder wasserdichte Kammer (10) für Schlüsselkomponenten (3) zum Betrieb der Boje eingebaut ist, wobei im Inneren der Kammer eine Batterie, der Prozessor, ein Positionierungsmodul zum Bestimmen der geografischen Position über ein oder mehrere Systeme zur globalen Navigation (GNSS, z. B. GALILEO, GPS, GLONAS, BEIDOU), Funk-WiFi-Sender und ein Empfänger sowie ein Modul zur Datenübertragung über Mobilfunknetze (z. B. LTE, 5G) installiert sind,
- das Unterteil (9) des Bojengehäuses unten eine Öffnung aufweist, durch die das UTP/Ethernet-Kabel (2) verläuft, und das Unterteil (9) ausgestaltet ist, um die Installation der beweglichen Kapsel (12) mit der Kamera (15) und Leuchten (16, 17) zu ermöglichen; oder das Gehäuse (9) im Unterteil als Hohlraum ausgebildet ist, der die Kapsel (12) mit der Kamera (15) und Leuchten (16, 17) zumindest teilweise umschließen kann;
- das UTP/Ethernet-Kabel (2), das die Kapsel (12) mit dem Bojengehäuse verbindet, das mit dem Prozessor verbunden ist, wobei sich das Kabel (2) mit Hilfe eines neben der Kammer (10) installierten Drehmechanismus ab- und aufwickelt, wobei der Drehmechanismus umfasst:
∘ eine Trommel, die an der Aluminiumwelle (6) montiert ist, die als Drehachse fungiert, und an der ein UTP-Kabel (2) installiert ist, wobei die Aluminiumwelle (6) mit Hilfe der Pylonen (19) im Gehäuse bis zu einem Gleitring (1) installiert ist;
o den Gleitring (1), der aus einem rotierendem und nicht rotierendem Teil besteht und angepasst ist, um einen elektrischen Kreis von der Batterie zur Kapsel (12) und ein Videosignal von der Kamera (15) zum Prozessor zu empfangen, wobei das UTP-Kabel (2) durch eine Aluminiumwelle (6) zum rotierenden Teil des Gleitrings (1) und über Schieber im Gleitring (1) zum nicht rotierenden Teil des Gleitrings, der in der Kammer (10) installiert ist, geführt wird;
∘ eine am Aluminiumrohr (6) angebrachte Buchse (5) oder angebrachtes Gleitlager zum Gewährleisten einer ungestörten Drehung des Rohres, und
∘ einziehbare Zugfedern (7), die das automatische Aufrollen und Aufwickeln des UTP-Kabels (2) auf der Trommel ermöglichen;
- ein Drahtseil (13), das am unteren Teil der Kapsel (12) befestigt ist und der freie Teil des Drahtseils (13) mit einem Ring zum Befestigen eines Ankers (S) oder eines anderen Gegenstands (D) oder einer Person ausgestattet ist (P).

2. Boje nach Anspruch 1, **dadurch gekennzeichnet, dass** das Positionierungsmodul ein oder mehrere Systeme für globale Navigation (GNSS) ist, ausgewählt aus einer Gruppe bestehend aus GALILEO, GPS, GLONAS, BEIDOU und einer beliebigen Kombination davon.

3. Boje nach einem der vorstehenden Ansprüche, **dadurch gekennzeichnet, dass** das Gehäuse (8, 9) der Boje aus ökologisch verarbeiteter, fester und widerstandsfähiger Kunststoffmasse besteht und dass beide Teile (8, 9) des Gehäuses mit Gewinden und Dichtungen zusammengebaut sind.

4. Boje nach einem der vorstehenden Ansprüche, **dadurch gekennzeichnet, dass** das Oberteil (8) der Boje teilweise transparent ist und zusätzlich Signalleuchten für den Fall einer Gefahr oder einer Bewegung des Ankers einschließen kann, und dass Solarzellen (18) am Gehäuse montiert sein können, wobei die Solarzellen (18) vorzugsweise im Inneren des Gehäuses unterhalb eines transparenten Materials des Gehäuseoberteils (8) eingebaut sind.

5. Boje nach einem der vorstehenden Ansprüche, **dadurch gekennzeichnet, dass** die Kapsel (12) vom Bojengehäuse (8, 9) durch Abwickeln des Kabels (2) mit dem Drehmechanismus, der durch Befestigen an dem Gewicht auf dem Drahtseil (13) und dadurch, dass sich die Kapsel (12) aufgrund der Schwerkraft nach unten bewegt, indem sie das Kabel (2) abwickelt, getrennt werden kann, während bei Entfernen des Gewichts auf dem Drahtseil (13) das Kabel (2) aufgrund der Funktion der Zugfeder (7), die fest am Ende der Aluminiumwelle (6) befestigt ist, an den beiden Pylonen (19) befestigt ist und zusammen mit der Buchse (5) eine stabile und sichere Befestigung der Trommel im Inneren des Bojengehäuses ermöglicht, automatisch auf die Trommel gewickelt wird, und die Buchse (6) eine Drehung der Welle ohne Zugkraft ermöglicht, und das Ende der Aluminiumwelle am Gleitlager befestigt ist, und das andere Teil fest an der einziehbaren Feder (7) mit konstanter Kraft montiert ist, die aus einem gewickelten Stahlband hergestellt ist, und die Windungen der Feder eine Dehnung und automatische Kontraktion ermöglichen.

6. Boje nach einem der vorstehenden Ansprüche, **dadurch gekennzeichnet, dass** die wasserundurchlässige und/oder wasserdichte Kammer (10) für Schlüsselkomponenten für die Bojenfunktion im Inneren des Bojengehäuses installiert ist und die Kammer (10) für Schlüsselkomponenten überwiegend aus zwei Teilen besteht und das Unterteil (11) mit Schrauben im unteren Teil (9) des Bojengehäuses festgeschraubt ist, und die Abdeckung mit Schrauben und Dichtungen fest am Unterteil der Kammer mit den wichtigsten Komponenten, wie der Batterie, dem Prozessor, dem Positionierungsmodul zum Bestimmen der geografischen Position über ein oder mehrere Systeme GALILEO, GPS und/oder GLONAS, Funk-WiFi-Sender und Empfänger und einem Modul zur Datenübertragung über Mobilfunknetze (LTE, 5G) befestigt ist, und das innere Teil von der Seite der Kammerabdeckung eine Öffnung aufweist, die einen nicht rotierenden Teil des Gleitrings (1) aufweist, der das UTP-Kabel (2) vom Prozessor über den Drehmechanismus zur Kapsel (12) führt.

7. Boje nach einem der vorstehenden Ansprüche, **dadurch gekennzeichnet, dass** die wasserundurchlässige Kapsel (12) aus dem Oberteil und dem Unterteil der Kapsel (12) besteht, die mit Schrauben und einer Dichtung zusammengebaut sind, wobei das Unterteil eine geeignet geformte Öffnung und ein versiegeltes transparentes Teil aufweist, damit die Kamera (15) Bilder aufnehmen kann, während die Leuchten (16, 17) Licht durch das transparente Material emittieren.

8. Boje nach einem der vorstehenden Ansprüche, **dadurch gekennzeichnet, dass** die gesamte Kapsel (12) mit Ausnahme der Öffnung für die Kamera (15) und Leuchten (16, 17) mit Gummi überzogen ist, das Stöße absorbiert und den Kunststoffteil der Kapsel (12) vor äußeren Einflüssen schützt.

9. Boje nach einem der vorstehenden Ansprüche, **dadurch gekennzeichnet, dass** sie angeordnet ist, um den Prozessor und die Anwendung zum Steuern der Kamera (15) über das UTP-Kabel (2) zu verwenden, wobei die Kamera (15) eine visuelle Steuerung/Überwachung eines gewissen Gegenstands (S, D) oder einer Person (P) unter Wasser in Echtzeit ermöglicht, wobei alle Aufnahmen und Bilder im Prozessor, der Anwendung und auf einem Server archiviert werden können.

10. Boje nach einem der vorstehenden Ansprüche, **dadurch gekennzeichnet, dass** die Kamera drei Funktionsmodi aufweist:
- wenn die LED-Leuchten (17) eingeschaltet sind, funktioniert die Kamera (15) normal,
- wenn nur die IR-Leuchten (16) eingeschaltet sind, funktioniert die Kamera (15) in einem Nachtmodus,
- Die Kamera (15) funktioniert auch bei ausgeschalteten Leuchten, sofern die Sichtverhältnisse geeignet sind und genügend natürliches Licht verfügbar ist.

11. Boje nach einem der vorstehenden Ansprüche, **dadurch gekennzeichnet, dass** die Anwendung mindestens die folgenden Schritte durchführt:
- Zeigt eine Video- oder Bildansicht des Ankers (S) an,
- zeigt die Position des Ankers (S) auf der Karte an,
- ermöglicht das Anpassen der Lichtverhältnisse (16, 17) und der Kamera (15) an die Boje wie oben beschrieben,
- ermöglicht das Geofencing des Ankers (S) bis zu 1 bis 20 Meter, was bedeutet, dass, wenn der Anker (S) von den voreingestellten Grenzen abweicht, der Alarm ausgelöst und der Benutzer benachrichtigt wird, dass sich der Anker außerhalb des gewissen Bereichs bewegt hat, wobei der Alarm akustisch, ein Bild sein oder auf andere Art und Weise erfolgen kann, und
- archiviert die Bild- und Videoinhalte durch Speichern auf dem geeigneten Server.

12. Boje nach einem der vorstehenden Ansprüche, **dadurch gekennzeichnet, dass** die Anwendung so angeordnet ist, dass sie zur bevorzugten Verwendung der Boje zum Überwachen des Zustands des Ankers (S) und zum Überprüfen der Verankerung (S) am selben Ort dient, wobei die Anwendung verwendet wird, um eine Geolokalisierung festzulegen, d. h. einen Kreis mit einem Radius von 1 bis 20 Metern zu definieren, wobei in dem Moment, in dem die Position festgelegt wird, die Position durch das Positionierungsmodul archiviert wird und die Einzäunung durch eine Software mit dem Prozessor implementiert, indem ein Begrenzungskreis auf der Karte gezeichnet wird, wobei jede mögliche zusätzliche Bewegung der Boje durch das Positionierungsmodul nach dem Festlegen der Position überwacht wird und wobei, wenn der Anker die voreingestellte Kreisgrenze überschreitet, der Alarm ausgelöst wird, wobei der Prozessor angeordnet ist, um entsprechend zu handeln.

13. Verfahren zum Verwenden der Boje nach einem der vorstehenden Ansprüche, wobei das Verfahren die folgenden Schritte umfasst:
a) Registrierung in einer Anwendung mit Benutzername und Passwort;
b) Verbinden mit der Boje oder einer spezifischen Boje, falls es mehrere gibt, und Benennen der Boje;
c) Anzeigen des Standorts der Boje auf der Karte;
d) Anschließen eines Ankers (S) oder eines an der Boje befestigten Gegenstands (D) oder einer Person (P) und Abtauchen in das Wasser, wodurch das Kabel automatisch von der Boje herabgelassen oder abgewickelt wird;
e) Sobald der Anker (S), der Gegenstand (D) oder die Person (P) auf dem Meeresboden oder in einer gewissen Tiefe platziert ist, stoppt das Abwickeln oder er/sie/es rollt zurück und senkt sich ggf. über ein Selbstaufrollsystem weiter ab, abhängig von den Wellen oder der Bewegung des Ankers oder des Gegenstands;
f) optional, Aktivierung von Licht und Aufnahme von Bildern oder Videos eines mit der Boje verbundenen Objekts;
g) Anzeigen von Bildern oder Videos mit der Option, Leuchten (IR, LED und Intensität) wie oben beschrieben zu verwalten und anzupassen;
h) optional die Möglichkeit, Bilder auf dem Smartphone zu bearbeiten und zu archivieren und auf die Galerie zuzugreifen;
i) optionale Festlegung geografischer Grenzen (Geofencing) von 1 bis 20 Metern, indem ein Kreis um die Bojenposition gezeichnet wird;
j) mögliche Alarmauslösung, wenn sich der Anker aus dem festgelegten Kreis herausbewegt;
k) optional, Ausschalten einer Kamera (15) und/oder der Leuchten (16, 17);
l) optional, Anheben des Ankers oder Gegenstands vom Meeresboden in Richtung Meeresoberfläche, was durch automatisches Aufwickeln des UTP-Kabels (2) mit Hilfe des Selbstaufrollsystems (7) erreicht wird; und
m) Heben der Boje aus dem Wasser.

## Revendications

1. Une bouée permettant de vérifier l'état d'une personne ou d'un objet attaché à ladite bouée, celle-ci comprenant :
- une enveloppe (8, 9), qui est conçue pour flotter sur la surface de la mer, et
- une capsule (12) équipée d'une caméra (15) et de lampes (16, 17), qui peut être séparée de l'enveloppe (8, 9) avec un câble UTP (2) et est conçue pour être descendue en direction d'une ancre, d'un objet ou d'une personne, où le câble (2) se déroule et s'enroule par le biais d'un mécanisme rotatif, qui comprend une bague coulissante (1) possédant une partie pivotante et une partie non-pivotante qui permettent le déroulement du câble UTP (2), qui assure le transfert sans faille de l'énergie électrique et du signal vidéo via le câble UTP (2) depuis une caméra (15) jusqu'à un compartiment imperméable et/ou étanche (10) pour le logement des composants clés (3) et inversement,
où la caméra (15) est conçue pour prendre des photos et/ou des vidéos et envoyer les données via le câble UTP (2) à un processeur, ledit processeur étant conçu pour envoyer des données par le biais d'un réseau sans fil (WiFi ou similaire) à une application mobile installée sur l'appareil de l'utilisateur, qui est de préférence un smartphone, une tablette, un ordinateur ou un appareil similaire,
**caractérisée par le fait que** :
- l'enveloppe (8, 9) comprend :
∘ une partie supérieure (8) ayant la forme d'une pyramide et étant pourvue de cellules photovoltaïques (18) pour fournir de l'énergie permettant le fonctionnement, et
∘ une partie inférieure (9) ayant la forme d'une pyramide tronquée ;
- à l'intérieur de l'enveloppe (8,9) est installé le compartiment imperméable et/ou étanche (10) pour les composants clés (3) permettant le fonctionnement de la bouée, où à l'intérieur du compartiment, une batterie, un processeur, un module de positionnement pour la détermination de la position géographique par le biais d'un ou plusieurs systèmes mondiaux de navigation par satellite (GNSS, p.ex. GALILEO, GPS, GLONAS, BEIDOU), un émetteur et un récepteur de signal WiFi et un module de transfert de données par le biais d'un réseau de communication mobile (p.ex. LTE, 5G) sont installés ;
- la partie inférieure (9) de l'enveloppe de la bouée possède une ouverture sur le dessous, par laquelle le câble UTP/Ethernet (2) passe, et la partie inférieure (9) est conçue pour permettre l'installation de la capsule amovible (12) équipée de la caméra (15) et des lampes (16, 17) ; ou alors l'enveloppe (9) est dans sa partie inférieure conçue comme une cavité, qui peut au moins partiellement contenir la capsule (12) équipée de la caméra (15) et des lampes (16, 17) ;
- le câble UTP/Ethernet (2), qui raccorde la capsule (12) à l'enveloppe de la bouée, et est raccordé au processeur, où le câble (2) se déroule et s'enroule à l'aide d'un mécanisme rotatif installé de façon contiguë au compartiment (10), ledit mécanisme rotatif comprenant :
∘ un enrouleur, installé sur une tige d'aluminium (6), qui fait office d'axe de rotation, sur lequel est installé un câble UTP (2), où la tige en aluminium (6) est installée dans l'enveloppe au moyen de pylônes (19) jusqu'à la bague coulissante (1) ;
∘ la bague coulissante (1), qui se compose d'une partie rotative et d'une partie non rotative, et qui est disposée de façon à recevoir un cercle électrique allant de la batterie jusqu'à la capsule (12) et un signal vidéo provenant de la caméra (15) et dirigé vers le processeur, où le câble UTP (2) est installé au moyen de la tige en aluminium (6) à la partie rotative de la bague coulissante (1), et par le biais de glisseurs présents dans la bague coulissante (1) à la partie non rotative de la bague coulissante, installée dans le compartiment (10) ;
∘ une douille (5) ou un palier coulissant installée sur la tige en aluminium (6) pour garantir une rotation fluide de ladite tige, et
∘ des ressorts de traction rétractables (7), qui permettent le déroulement et l'enroulement automatique du câble UTP (2) sur l'enrouleur ;
- un câble métallique (13) fixé sur la partie inférieure de la capsule (12), et la partie libre du câble métallique (13) est équipée d'un cerceau pour se fixer à une ancre (S) ou tout autre objet (D) ou personne (P).

2. La bouée selon la revendication 1, **caractérisée par le fait que** le module de positionnement est un ou plusieurs systèmes mondiaux de navigation par satellite (GNSS), sélectionné dans le groupe comprenant GALILEO, GPS, GLONAS, BEIDOU et toute combinaison de ceux-ci.

3. La bouée selon l'une quelconque des revendications précédentes, **caractérisée par le fait que** l'enveloppe (8, 9) de la bouée est constituée d'une matière plastique résistante, robuste et produite de façon écologique, et **par le fait que** les deux parties (8,9) de l'enveloppe sont assemblées avec des fils et des joints d'étanchéité.

4. La bouée selon l'une quelconque des revendications précédentes, **caractérisée par le fait que** la partie supérieure (8) de la bouée est partiellement transparente et peut en outre comprendre des feux de signalisation en cas de danger ou de mouvement de l'ancre, et **par le fait que** les cellules photovoltaïques (18) peuvent être installées sur l'enveloppe, de préférence les cellules photovoltaïques (18) sont installées à l'intérieur de l'enveloppe sous le matériau transparent de la partie supérieure (8) de l'enveloppe.

5. La bouée selon l'une quelconque des revendications précédentes, **caractérisée par le fait que** la capsule (12) peut être séparée de l'enveloppe de la bouée (8, 9) en déroulant le câble (2) avec le mécanisme rotatif, qui agit en se fixant au poids du câble métallique (13), et en raison de la gravité, la capsule (12) se déplace vers le bas en déroulant le câble (2), alors que quand le poids est retiré du câble métallique (13), le câble (2) s'enroule automatiquement sur l'enrouleur en raison de l'action du ressort de traction (7), qui est fixé à l'extrémité de la tige en aluminium (6), elle-même fixée aux deux pylônes (19), et permet, à l'aide de la douille (5), à l'enrouleur d'être stable et fixé à l'intérieur de l'enveloppe de la bouée, et la douille (6) permet la rotation de la tige sans traction, et l'extrémité de la tige en aluminium est fixée au palier coulissant, et l'autre partie est fixée au ressort rétractable (7) avec une force constante, celui-ci étant constitué d'une spirale de feuillard en acier, et la spirale du ressort permet l'étirement et la contraction automatiques.

6. La bouée selon l'une quelconque des revendications précédentes, **caractérisée par le fait que** le compartiment imperméable et/ou étanche (10) pour les composants clés du fonctionnement de la bouée est installé à l'intérieur de l'enveloppe de la bouée, et pour l'essentiel le compartiment (10) pour les composants clés est constitué de deux parties, et la partie inférieure (11) dotée de vis est enroulée dans la partie inférieure (9) de l'enveloppe de la bouée, et le couvercle est fixé hermétiquement avec des vis et des joints d'étanchéité à la partie inférieure du compartiment des composants clés tels que la batterie, le processeur, le module de positionnement pour déterminer la position géographique par le biais d'un ou plusieurs systèmes GALILEO, GPS, et/ou GLONAS, un émetteur et récepteur de signal WiFi et un module pour le transfert des données via des réseaux de communication mobile (LTE, 5G), et la partie intérieure près du côté du couvercle du compartiment possède une ouverture, qui a une partie non-rotative de la bague coulissante (1), qui fait passer le câble UTP (2) depuis le processeur via le mécanisme de rotation jusqu'à la capsule (12).

7. La bouée selon l'une quelconque des revendications précédentes, **caractérisée par le fait que** la capsule étanche (12) est composée d'une partie supérieure et d'une partie inférieure (12), qui sont assemblées avec des vis et un joint d'étanchéité, où la partie inférieure possède une ouverture de conception appropriée et une partie transparente étanche pour que la caméra (15) puisse prendre des photos, tandis que les lampes (16, 17) émettent de la lumière à travers le matériau transparent.

8. La bouée selon l'une quelconque des revendications précédentes, **caractérisée par le fait que** l'intégralité de la capsule (12), sauf l'ouverture pour la caméra (15) et les lampes (16, 17), est recouverte de caoutchouc qui absorbe les chocs et protège la partie en plastique de la coque de la capsule (12) des facteurs extérieurs.

9. La bouée selon l'une quelconque des revendications précédentes, **caractérisée par le fait qu'**elle est conçue de façon à utiliser le processeur et l'application pour contrôler la caméra (15) par le biais du câble UTP (2), ladite caméra (15) permettant le contrôle/suivi visuel d'un certain objet (S, D) ou d'une personne (P) sous l'eau en temps réel, où tous les enregistrements et images peuvent être archivés dans le processeur, l'application et sur un serveur.

10. La bouée selon l'une quelconque des revendications qui précèdent, **caractérisée par le fait que** la caméra a trois modes de fonctionnement :
- si les lampes LED (17) sont allumées, la caméra (15) fonctionne normalement,
- si seules les lampes IR (16) sont allumées, la caméra (15) fonctionne en mode nocturne,
- la caméra (15) fonctionne même quand les lumières sont éteintes, si la visibilité est appropriée et qu'il y a suffisamment de lumière naturelle.

11. La bouée selon l'une quelconque des revendications qui précèdent, **caractérisée par le fait que** l'application effectue au moins les étapes suivantes :
- affiche une visualisation vidéo ou en images de l'ancre (S),
- affiche la localisation de l'ancre (S) sur la carte,
- permet d'ajuster la lumière (16, 17) et la caméra (15) sur la bouée comme décrit ci-dessus,
- permet le gardiennage virtuel de l'ancre (S) de 1 à 20 mètres, ce qui signifie que si l'ancre (S) dépasse les limites pré-établies, l'alarme se déclenche, prévenant l'utilisateur que l'ancre est passée en dehors de la zone définie, l'alarme pouvant être sonore, visuelle ou de tout type approprié et
- elle archive les images et les vidéos en les stockant sur le serveur approprié.

12. La bouée selon l'une quelconque des revendications précédentes, **caractérisée par le fait que** l'application est conçue de façon à ce que pour l'utilisation préférée de la bouée dans le suivi de l'état de l'ancre (S) et le gardiennage de l'ancre (S) au même endroit, l'application est utilisée pour définir un emplacement géographique, établissant ainsi un cercle d'un rayon allant de 1 à 20 mètres, où à partir du moment où la position est définie, celle-ci est archivée par le module de positionnement, et le gardiennage est mis en oeuvre par un logiciel avec le processeur, traçant ainsi un cercle de délimitation sur la carte, où tout mouvement ultérieur potentiel de la bouée est suivi par le module de positionnement après la configuration de la position, et où dans le cas où l'ancre franchit la limite que constitue le cercle prédéfini, l'alarme est conçue pour être déclenchée, où le processeur est conçu pour agir en conséquence.

13. Un procédé d'utilisation de la bouée selon l'une quelconque des revendications précédentes, ledit procédé comprenant les étapes suivantes :
a) l'enregistrement dans une application avec un nom d'utilisateur et un mot de passe ;
b) la connexion à la bouée ou à une bouée donnée, s'il y en a plusieurs, et nommage de la bouée ;
c) l'affichage de la localisation de la bouée sur la carte ;
d) la connexion à une ancre (S) ou un objet (D) ou une personne (P) attachée à la bouée et descente dans l'eau, ce qui provoque la descente ou le déroulement automatique du câble depuis la bouée ;
e) une fois l'ancre (S) ou l'objet (D) ou la personne (P) placée sur le fond marin ou à une certaine profondeur, le déroulement s'arrête ou repart en sens inverse puis redescend au moyen du système d'enroulement automatique si besoin est, en fonction des vagues ou du mouvement de l'ancre ou de l'objet ;
f) facultativement, l'activation de la lumière et la saisie d'images ou d'une vidéo de l'objet relié à la bouée ;
g) l'affichage de l'image ou de la vidéo avec la possibilité de gérer et d'ajuster les lampes (IR, LED et de haute intensité) ainsi que décrit ci-dessus ;
h) facultativement, la possibilité d'améliorer et d'archiver les images sur le smartphone et accès à la galerie ;
i) définition facultative de limites géographiques (gardiennage virtuel) allant de 1 à 20 mètres, en traçant un cercle autour de la position de la bouée ;
j) déclenchement possible de l'alarme quand l'ancre franchit la limite du cercle défini ;
k) facultativement, extinction de la caméra (15) et/ou des lampes (16, 17) ;
l) facultativement, levage de l'ancre ou de l'objet du fond marin vers la surface de l'eau, ce qui est réalisé en enroulant automatiquement le câble UTP (2) à l'aide du système d'enroulement automatique (7) ; et
m) extraction de la bouée hors de l'eau.
